# EUROPÄISCHE PATENTSCHRIFT

(11) **EP 2 078 013 B1**
(45) Veröffentlichungstag und Bekanntmachung des Hinweises auf die Patenterteilung: **04.09.2013**
(21) Anmeldenummer: 07818207.8
(22) Anmeldetag: 18.09.2007
(51) Int. Cl.: C07D 405/12

(54) **NEUE KRISTALLINE MODIFIKATION VON 4- (N-METHYL-2-CHLORO-5-PYRIDYLMETHYLAMINO) -2,5-DIHYDROFURAN-2-ON**
NOVEL CRYSTALLINE MODIFICATION OF 4- (N-METHYL-2-CHLORO-5-PYRIDYLMETHYLAMINO) -2,5-DIHYDROFURAN-2-ONE
NOUVELLE MODIFICATION CRISTALLINE DE 4- (N-MÉTHYL-2-CHLORO-5-PYRIDYLMÉTHYLAMINO) -2,5-DIHYDROFURAN-2-ONE

(30) Priorität: 29.09.2006 DE 102006046161
(43) Veröffentlichungstag der Anmeldung: 15.07.2009
(73) Patentinhaber: Bayer CropScience AG, 40789 Monheim (DE)
(72) Erfinder: OLENIK, Britta, 46242 Bottrop (DE); JESCHKE, Peter, 51467 Bergisch Gladbach (DE); VELTEN, Robert, 40764 Langenfeld (DE); GALLENKAMP, Bernd, 42113 Wuppertal (DE); JOERGES, Wolfgang, 51519 Odenthal (DE); VERMEER, Ronald, 51371 Leverkusen (DE); PITTA, Leonardo, 51371 Leverkusen (DE)
(86) Internationale Anmeldenummer: PCT/EP2007/008102
(87) Internationale Veröffentlichungsnummer: WO 2008/040445

(56) Entgegenhaltungen:
- EP-A- 0 539 588

## Beschreibung

Die vorliegende Erfindung betrifft eine neue kristalline Modifikation der Verbindung der Formel (I), Verfahren zu ihrer Herstellung und ihre Verwendung in agrochemischen Zubereitungen.

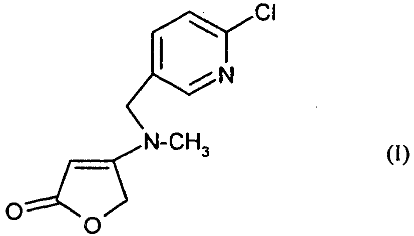

Die Verbindung der Formel (I) aus EP-A 0 539 588 ist bekannt. Sie kann durch die dort beschriebenen Verfahren synthetisiert werden.

Bei dem vorbeschriebenen Syntheseverfahren wird die Verbindung der Formel (I) der kristallinen Modifikation II (zur Nomenklatur und Charakterisierung siehe weiter unten in der Beschreibung) erhalten. Diese kristalline Modifikation ist metastabil.

Es ist bekannt, dass für einige Polymorphe eine bestimmte Modifikation über den gesamten Temperaturbereich bis zum Schmelzpunkt die thermodynamisch stabile Phase darstellt, wohingegen bei anderen Stoffsystemen ein oder mehrere Übergangspunkte existieren, bei dem sich das Stabilitätsverhältnis umkehrt. Es ist nicht möglich, das Stabilitätsverhältnis und insbesondere die Existenz und Lage von oben bezeichneten Übergangspunkten vorherzusagen. Ein Überblick über den Stand des Wissens zu diesen prinzipiellen thermodynamischen Verhältnissen ist in J. Bernstein, R.J. Davey, J.O. Henck, Angew. Chem. Int. Ed., 1999, 38, 3440-3461 gegeben.

Das Auftreten von Wirkstoffen in verschiedenen kristallinen Modifikationen (= Polymorphie) ist sowohl für die Ausarbeitung von Herstellungsverfahren als auch für die Entwicklung von Formulierungen von großer Bedeutung. So unterscheiden sich die verschiedenen kristallinen Modifikationen einer chemischen Verbindung neben dem Aussehen (Kristallhabitus) und der Härte auch in zahlreichen weiteren physiko-chemischen Eigenschaften. Dabei können Unterschiede bezüglich der Stabilität, der Filtrierbarkeit, der Mahlbarkeit, der Löslichkeit, der Hygroskopizität, des Schmelzpunktes, der Feststoffdichte und der Fließfähigkeit einen starken Einfluss auf die Qualität und die Wirksamkeit von Pflanzenbehandlungsmitteln ausüben. Es ist bisher nicht möglich, das Auftreten und die Anzahl von kristallinen Modifikationen einschließlich ihrer physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Metastabile Kristallmodifikationen, wie die kristalline Modifikation II der Verbindung der Formel (I) haben generell Nachteile im Vergleich zu einer thermodynamisch stabilen Form bezüglich des Herstellprozesses, sowie bei der Lagerung und beim Transport der Wirkstoffe und Formulierungen. Aus J. Halebian, W. McCrone, J. Pharm. Sci. 58 (1969) 911 ist bekannt, dass beim Einsatz einer thermodynamisch metastabilen Form bei der Herstellung oder Lagerung eine vollständige oder teilweise Umwandlung in eine andere polymorphe Form stattfinden kann. Als Begleiterscheinung wird dabei unerwünschtes Kristallwachstum (Rekristallisation), Veränderungen in der Bioverfügbarkeit, Agglomeration usw. beobachtet. Die Umwandlung kann dabei über einen längeren Zeitraum oder spontan erfolgen und kann nicht vorhergesagt werden. Ob, wann, und in welcher Menge eine andere Kristallmodifikation entsteht, bleibt weitgehend dem Zufall überlassen. Dieses Verhalten metastabiler Kristallmodifikationen kann einen großen Einfluss auf die Entwicklung, den Transport und insbesondere auf die Lagerstabilität haben.

Weiterhin ist es bekannt, dass metastabile Kristallmodifikationen, wie die kristalline Modifikation II der Verbindung der Formel (I), bei der Herstellung von Formulierungen, insbesondere solchen Formulierungen in denen der Wirkstoff in fester Form vorliegt, oder in den Fällen in denen der Wirkstoff während der Herstellung der Formulierungen eine gewisse Zeit in fester Form vorliegt negative Eigenschaften der Formulierung hervorrufen können. Es ist sogar nicht auszuschließen, dass die Verwendung von metastabilen Kristallmodifikationen die Herstellung von Formulierungen unmöglich macht. Bei der Herstellung von Formulierungen, bei denen der Wirkstoff in fester Form eingesetzt wird, muss im Allgemeinen der Wirkstoff zerkleinert werden. Dieser Prozess ist mechanisch und verursacht deshalb eine Steigerung der Temperatur. Eine thermodynamisch metastabile Form des Wirkstoffs wird durch eine Steigerung der Temperatur die Tendenz zeigen in die stabile Modifikation umzuwandeln, was zur Festmahlung führen kann. Unter Festmahlung ist zu verstehen, dass sich der Wirkstoff bzw. die Wirkstoffsuspension und die Mahlkörper im Mahlgerät bis zur kompletten Aushärtung verfestigen. Das Risiko einer Festmahlung wird durch die nicht vorhersagbare Umwandlungsgeschwindigkeit der metastabilen Modifikation beeinflusst. Auch wenn die Formulierungen mit einem Wirkstoff der metastabilen Modifikation herstellbar sind, ist oft die Lagerung der Formulierungen problematisch weil hier auch eine sehr niedrige Umwandlungsgeschwindigkeit der metastabilen Modifikation die Lagerungseigenschaften negativ beeinflusst. Beispielhafte negative Eigenschaften, welche durch eine solche Umwandlung während der Lagerung verursacht werden können sind Kristallwachstum, Agglomeration der Teilchen, Sedimentation oder Verfestigen des Produkts.

Der Erfindung liegt daher die Aufgabe zugrunde, eine neue kristalline Modifikation der Verbindung der Formel (I) herzustellen, die aufgrund ihrer physiko-chemischen Eigenschaften in Formulierungen gut zu handhaben ist. Eine weitere Aufgabe der vorliegenden Erfindung ist die Herstellung einer neuen kristallinen Modifikation der Verbindung der Formel (I), die zur Herstellung von Formulierungen, die eine Verwendung von Mahlprozessen erfordern, besonders gut geeignet ist. Beispielhaft und vorzugsweise - ohne jedoch zu limitieren - seien die folgenden Formulierungen genannt: Formulierungen, in denen der Wirkstoff in fester (trockener) Form vorliegt wie z.B.: Granulate, verkapselte Granulate, Tabletten, wasserdispergierbare Granulate, wasserdispergierbare Tabletten, wasserdispergierbare Pulver oder wasserdispergierbare Pulver für Saatgutbehandlung, Staub-Formulierungen; Formulierungen, in denen der Wirkstoff in dispergierter Form vorliegt wie z.B.: Suspensionskonzentrate, Öl basierte Suspensionskonzentrate, Suspo-Emulsionen, oder Suspensionskonzentrate für Saatgutbehandlung.

Diese Aufgabe wird erfindungsgemäß durch eine neue kristalline Modifikation der Verbindung der Formel (I) gelöst, die nachstehend als kristalline Modifikation I (stabile Modifikation) bezeichnet wird.

Gegenstand der Erfindung ist daher die kristalline Modifikation I, die dadurch gekennzeichnet ist, dass sie ein Röntgen-Pulverdiffraktogramm bei Verwendung von Cu Kα-Strahlung mit folgenden Reflexlagen (2 Theta, > 20% relative Intensität) aufweist:

| **2Theta** / ° |
|---|
| 16.80 |
| 19.58 |
| 21.11 |
| 21.32 |
| 23.23 |
| 28.00 |

Gemäß einer bevorzugten Ausführungsform ist die kristalline Modifikation I dadurch gekennzeichnet, dass sie ein Röntgen-Pulverdiffraktogramm bei Verwendung von Cu Kα-Strahlung mit den folgenden Reflexlagen (2 Theta, > 20% relative Intensität) aufweist:

| **2Theta** / ° |
|---|
| 16.80 |
| 19.58 |
| 21.11 |
| 21.32 |
| 22.92 |
| 23.23 |
| 23.97 |
| 28.00 |

Gemäß einer weiteren bevorzugten Ausführungsform ist die kristalline Modifikation I dadurch gekennzeichnet, dass sie ein Röntgen-Pulverdiffraktogramm bei Verwendung von Cu Kα-Strahlung mit den folgenden Reflexlagen (2 Theta, > 20% relative Intensität) aufweist:

| **2Theta** / ° |
|---|
| 8.07 |
| 15.16 |
| 16.80 |
| 19.58 |
| 20.03 |
| 21.11 |
| 21.32 |
| 22.92 |
| 23.23 |
| 23.97 |
| 24.46 |
| 24.54 |
| 27.26 |
| 27.60 |
| 28.00 |

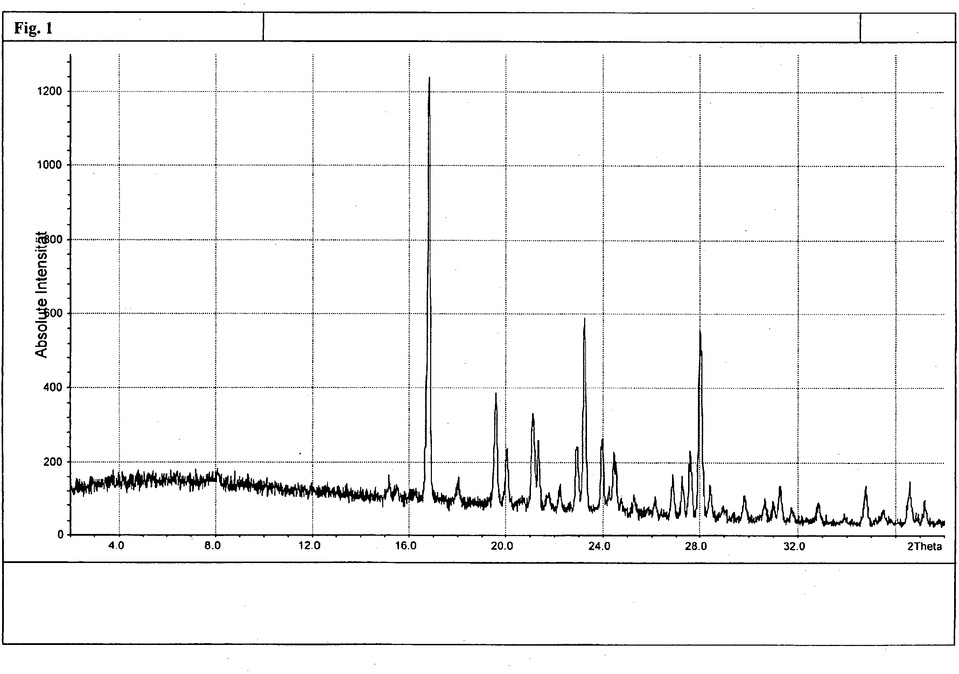

Gemäß einer besonders bevorzugten Ausführungsform der vorliegenden Erfindung weist das Röntgen-Pulverdiffraktogramm der kristallinen Modifikation I die in Tabelle 1 angegebenen Reflexlagen auf. Gemäß einer weiteren ganz besonders bevorzugten Ausführungsform der vorliegenden Erfindung weist das Röntgen-Pulverdiffraktogramm der kristallinen Modifikation I die in der Abbildung 1 wiedergegebenen Signale auf. Die intensivsten Signale (2 Theta) des Röntgen-Pulverdiffraktogramms der kristallinen Modifikation I liegen demnach bei 16.80°, 19.58°, 21.11 °, 21.32°, 22.92°, 23.23°, 23.97° und 28.00° (jeweils ± 0,2°).

Alle Röntgen-Pulverdiffraktometrie-Daten wurden mit folgenden Akquisitionsparametern erhalten:

| | |
|---|---|
| Diffraktometer: | Transmission |
| Monochromator: | Curved Germanium (111) |
| Wellenlänge: | 1.540598 Cu |
| Detektor: | Linear PSD |
| Scan Modus: | Transmission / Moving PSD / Fixed omega |
| Scan Typ: | 2Theta:Omega |
| Angabe 2Theta: | ± 0,2° |

**Tabelle 1**

| **2Theta /** ° | **Relative Intensität¹** |
|---|---|
| 8.07 | 14 |
| 15.16 | 13 |
| 15.51 | 11 |
| 16.25 | 9 |
| 16.80 | 100 |
| 18.02 | 12 |
| 19.58 | 30 |
| 20.03 | 18 |
| 20.67 | 8 |
| 21.11 | 26 |
| 21.32 | 21 |
| 21.69 | 9 |
| 21.77 | 9 |
| 22.19 | 9 |
| 22.92 | 20 |
| 23.23 | 48 |
| 23.97 | 20 |
| 24.26 | 11 |
| 24.46 | 17 |
| 24.54 | 14 |
| 24.76 | 8 |
| 25.31 | 8 |
| 25.88 | 6 |
| 26.14 | 7 |
| 26.87 | 12 |
| 27.26 | 13 |
| 27.60 | 18 |
| 28.00 | 45 |
| 28.41 | 11 |
| 28.96 | 6 |
| 29.09 | 6 |
| 29.80 | 9 |
| 30.65 | 7 |
| 31.00 | 7 |
| 31.29 | 10 |
| 31.77 | 6 |
| 32.86 | 6 |
| 33.91 | 5 |
| 34.78 | 10 |
| 35.47 | 6 |
| 35.78 | 4 |
| 36.57 | 10 |
| 36.86 | 3 |
| 37.16 | 7 |

| | |
|---|---|
| ¹Intensität relativ zum intensivsten Signal des Spektrums, das willkürlich mit 100 definiert wird. | |

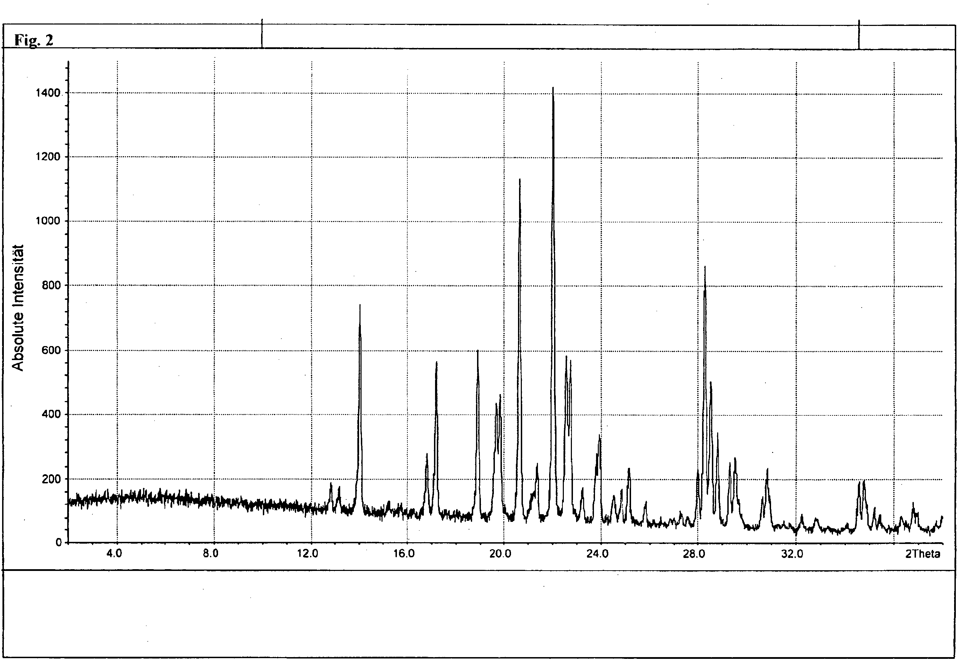

Die bekannte kristalline Modifikation II der Verbindung der Formel (I) ist dadurch gekennzeichnet, dass sie ein Röntgen-Pulverdiffraktogramm mit den in der folgenden Tabelle 2 angegebenen Reflexlagen (2 Theta) aufweist. Das Röntgen-Pulverdiffraktogramm der kristallinen Modifikation II ist auch in der Abbildung 2 wiedergegeben.

**Tabelle 2**

| **2Theta / °** | **Relative Intensität¹** |
|---|---|
| 12.82 | 14 |
| 13.17 | 12 |
| 14.02 | 52 |
| 15.23 | 9 |
| 15.75 | 7 |
| 16.81 | 19 |
| 17.19 | 38 |
| 18.90 | 42 |
| 19.66 | 31 |
| 19.81 | 33 |
| 20.01 | 8 |
| 20.63 | 81 |
| 21.19 | 11 |
| 21.34 | 17 |
| 22.01 | 100 |
| 22.55 | 41 |
| 22.71 | 40 |
| 22.93 | 8 |
| 23.22 | 11 |
| 23.79 | 18 |
| 23.92 | 24 |
| 24.54 | 11 |
| 24.83 | 12 |
| 25.16 | 17 |
| 25.81 | 8 |
| 26.18 | 5 |
| 26.44 | 4 |
| 26.84 | 6 |
| 27.01 | 5 |
| 27.33 | 7 |
| 27.59 | 6 |
| 27.97 | 16 |
| 28.26 | 62 |
| 28.52 | 36 |
| 28.79 | 24 |
| 29.31 | 18 |
| 29.53 | 18 |
| 29.67 | 9 |
| 29.79 | 7 |
| 30.62 | 10 |
| 30.82 | 16 |
| 31.30 | 4 |
| 31.53 | 5 |
| 31.78 | 5 |
| 32.23 | 6 |
| 32.83 | 6 |
| 33.33 | 4 |
| 34.10 | 4 |
| 34.57 | 13 |
| 34.78 | 13 |
| 35.21 | 7 |
| 35.40 | 6 |
| 36.31 | 6 |
| 36.49 | 4 |
| 36.79 | 9 |
| 36.95 | 7 |
| 37.71 | 4 |

| | |
|---|---|
| ¹Intensität relativ zum intensivsten Signal des Spektrums, das willkürlich mit 100 definiert wird. | |

Die erfindungsgemäße kristalline Modifikation I der Verbindung der Formel (I) kann außerdem durch IR- und Raman-Spektroskopie charakterisiert werden.

Ein weiterer Gegenstand der Erfindung ist daher die kristalline Modifikation I der Verbindung der Formel (I), die dadurch gekennzeichnet ist, dass sie ein IR Spektrum mit folgenden Banden [cm⁻¹] aufweist:

| **[cm⁻¹]** |
|---|
| 3122 |
| 1584 |
| 1055 |
| 933 |
| 896 |
| 800 |

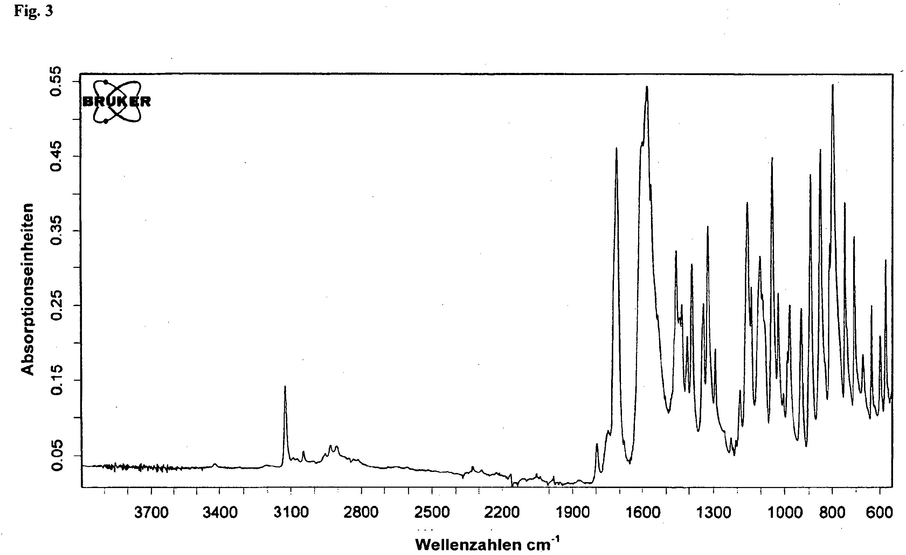

Gemäß einer bevorzugten Ausführungsform der vorliegenden Erfindung weist das IR Spektrum der kristallinen Modifikation I der Verbindung der Formel (I) die in Tabelle 3 angegebenen Banden auf. Gemäß einer weiteren besonders bevorzugten Ausführungsform der vorliegenden Erfindung weist das IR Spektrum der kristallinen Modifikation I die in der Abbildung 3 wiedergegebenen Signale auf.

Ein weiterer Gegenstand der Erfindung ist die kristalline Modifikation I der Verbindung der Formel (I), die dadurch gekennzeichnet ist, dass sie ein Raman Spektrum mit folgenden Banden [cm⁻¹] aufweist:

| **[cm⁻¹]** |
|---|
| 3047 |
| 1719 |
| 1601 |
| 1342 |
| 1254 |
| 936 |

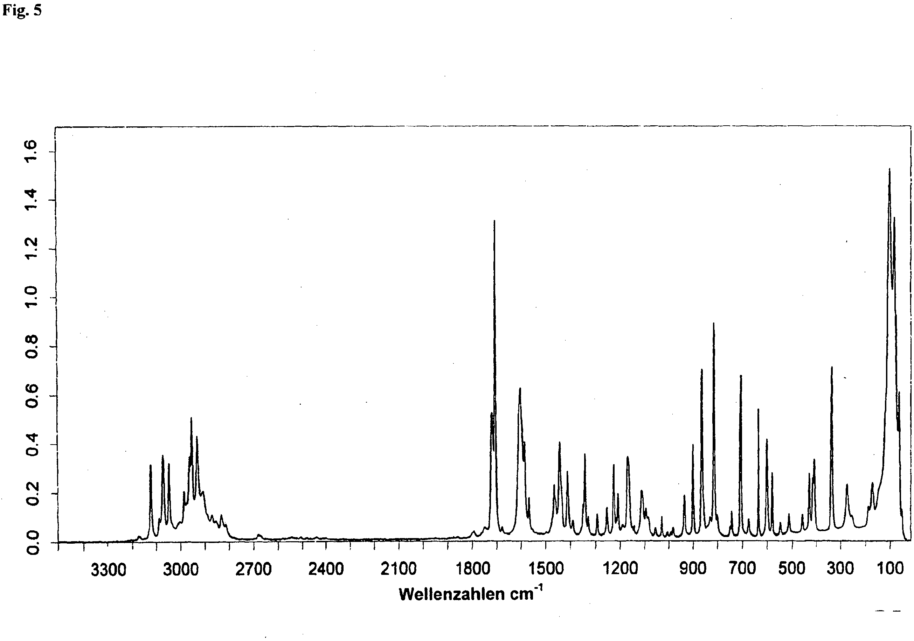

Gemäß einer bevorzugten Ausführungsform der vorliegenden Erfindung weist das Raman Spektrum der kristallinen Modifikation I der Verbindung der Formel (I) die in Tabelle 3 angegebenen Banden auf. Gemäß einer weiteren ganz besonders bevorzugten Ausführungsform der vorliegenden Erfindung weist das Raman Spektrum der kristallinen Modifikation I die in der Abbildung 5 wiedergegebenen Signale auf.

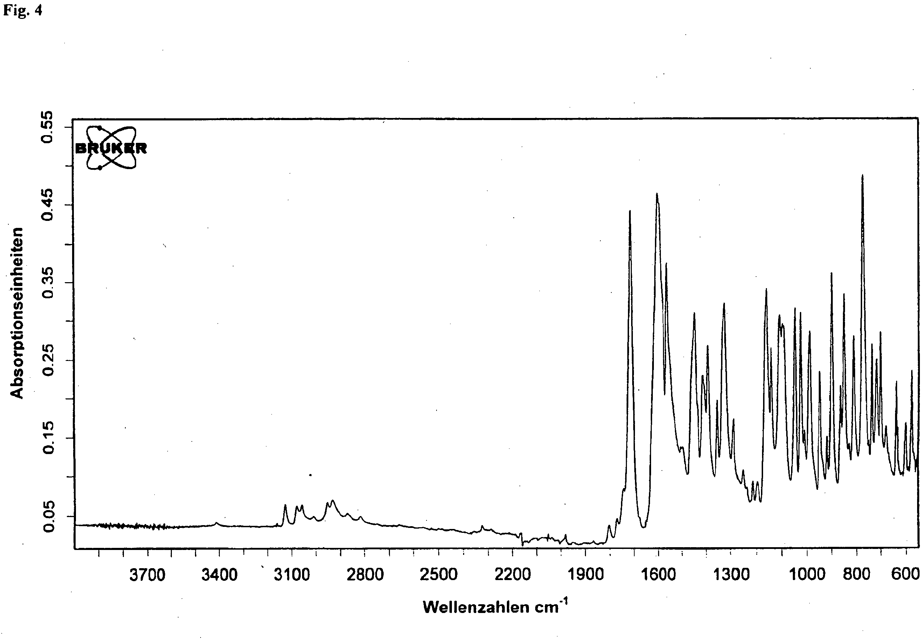

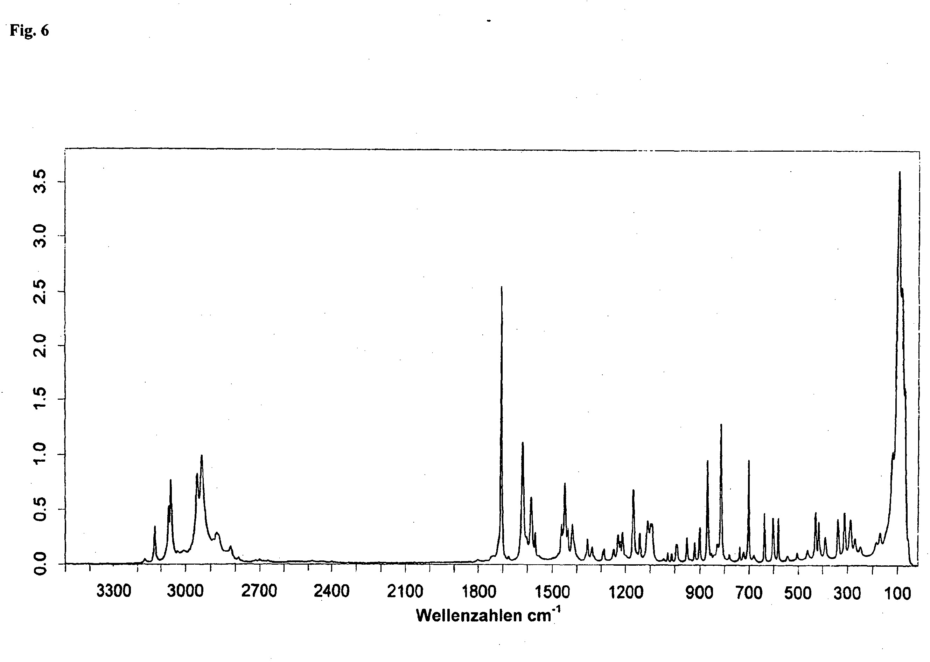

Die bekannte kristalline Modifikation II der Verbindung der Formel (I) ist dadurch gekennzeichnet, dass sie ein IR bzw. Raman Spektrum mit den in der folgenden Tabelle 3 angegebenen Banden aufweist. Das IR Spektrum der kristallinen Modifikation II ist auch in der Abbildung 4 und das Raman Spektrum der kristallinen Modifikation II ist auch in der Abbildung 6 wiedergegeben.

Alle IR Spektren wurden mit folgenden Aquisitionsparametern gewonnen:

| | |
|---|---|
| FT-IR-Spektrometer | Bruker Tensor 37 |
| Diamond ATR Einheit | Fa. Specac |
| Wellenlängenbereich | 550 - 4000 cm⁻¹ |
| Auflösung | 2 cm⁻¹ |
| Zahl der Scans | 64 |

Alle Raman Spektren wurden mit folgenden Aquisitionsparametern gewonnen:

| | |
|---|---|
| FT-Raman-Spektrometer | Bruker RFS/100 |
| Laser Wellenzahl | 15798.7 cm⁻¹ |
| Auflösung | 1 cm⁻¹ |
| Zahl der Scans | 128 |

**Tabelle 3**

| **I** | **II** | **I** | **II** |
|---|---|---|---|
| **IR-Banden [cm⁻¹]** | **IR-Banden [cm⁻¹]** | **Raman-Banden [cm⁻¹]** | **Raman-Banden [cm⁻¹]** |
| 800 | 720 | 335 | 183 |
| 813 | 737 | 409 | 249 |
| 854 | 749 | 415 | 270 |
| 896 | 776 | 429 | 288 |
| 933 | 814 | 457 | 312 |
| 981 | 830 | 513 | 337 |
| 990 | 852 | 547 | 387 |
| 1006 | 866 | 579 | 415 |
| 1030 | 901 | 601 | 428 |
| 1055 | 918 | 635 | 462 |
| 1106 | 948 | 675 | 503 |
| 1143 | 989 | 706 | 540 |
| 1159 | 1010 | 709 | 544 |
| 1189 | 1026 | 743 | 580 |
| 1227 | 1048 | 801 | 602 |
| 1294 | 1098 | 815 | 639 |
| 1326 | 1109 | 832 | 680 |
| 1343 | 1141 | 864 | 703 |
| 1390 | 1160 | 900 | 722 |
| 1409 | 1195 | 936 | 738 |
| 1434 | 1212 | 982 | 779 |
| 1443 | 1250 | 1030 | 814 |
| 1458 | 1288 | 1056 | 830 |
| 1567 | 1326 | 1095 | 853 |
| 1584 | 1354 | 1112 | 869 |
| 1713 | 1395 | 1142 | 900 |
| 1750 | 1415 | 1168 | 919 |
| 1797 | 1450 | 1208 | 950 |
| 2906 | 1505 | 1226 | 990 |
| 2930 | 1566 | 1254 | 993 |
| 3047 | 1607 | 1293 | 1012 |
| 3122 | 1716 | 1330 | 1029 |
| | 1802 | 1342 | 1046 |
| | 2816 | 1391 | 1092 |
| | 2871 | 1410 | 1098 |
| | 1932 | 1443 | 1110 |
| | 2953 | 1466 | 1141 |
| | 3010 | 1568 | 1167 |
| | 3058 | 1585 | 1212 |
| | 3078 | 1601 | 1222 |
| | 3126 | 1679 | 1233 |
| | | 1705 | 1250 |
| | | 1719 | 1288 |
| | | 1794 | 1293 |
| | | 2678 | 1338 |
| | | 2831 | 1355 |
| | | 2869 | 1418 |
| | | 2907 | 1449 |
| | | 2930 | 1464 |
| | | 2952 | 1568 |
| | | 2961 | 1584 |
| | | 2985 | 1619 |
| | | 3047 | 1706 |
| | | 3070 | 2816 |
| | | 3122 | 2873 |
| | | | 2934 |
| | | | 2952 |
| | | | 3057 |
| | | | 3067 |
| | | | 3126 |
| | | | 3168 |

Es wurde überraschend gefunden, dass die Verbindung der Formel (I) in der kristallinen Modifikation I thermodynamisch stabil ist und sich auch bei längerer Lagerung nicht in eine andere kristalline Modifikationen umwandelt. Es wurde außerdem gefunden, dass die kristalline Modifikation I im Vergleich mit anderen kristallinen Modifikationen sich besser Zerkleinern/Mahlen lässt und dass die mit dieser Modifikation hergestellten Formulierungen auch nach Lagerung stabil sind. Die Herstellung von Suspensionskonzentraten, Öl basierten Suspensionskonzentraten und z.B. wasserdispergierbaren Granulaten sowie ähnlicher Formulierungen zur Behandlung von Saatgut ist dadurch möglich geworden. Darüber hinaus neigt die kristalline Modifikation I im Vergleich zu der kristallinen Modifikation II weit weniger zur Aufnahme von Wasser aus der Luft. Sie ist aus diesen Gründen hervorragend zur Herstellung von Festformulierungen geeignet. Durch ihre Stabilität verleiht sie diesen Formulierungen die gewünschte lang andauernde Lagerstabilität. Mit der kristallinen Modifikation I können damit definiert und gezielt stabile feste Zubereitungen der Verbindung der Formel (I) hergestellt werden.

Die Verbindung der Formel (I) in der kristallinen Modifikation I eignet sich aufgrund ihrer Stabilität hervorragend für die Zubereitung von Mitteln zur Bekämpfung von Schädlingen. Gegenstand der Erfindung sind daher auch Mittel zur Bekämpfung von Schädlingen, welche die kristalline Modifikation I der Verbindung der Formel (I) alleine oder in Mischung mit Hilfs- und Trägerstoffen, sowie in Mischung mit anderen Wirkstoffen enthalten.

Die Erfindung schließt auch Zusammensetzungen ein, die die kristalline Modifikation I und die kristalline Modifikation II der Verbindung der Formel (I) enthalten. Bevorzugt sind Zusammensetzungen, die weniger als 20 Gew.-% der kristallinen Modifikation II der Verbindung der Formel (I), besonders bevorzugt weniger als 15 Gew.-%, ganz besonders bevorzugt mit weniger als 10 Gew.-%, insbesondere bevorzugt mit weniger als 5 Gew. %, am meisten bevorzugt mit weniger als 4, 3, 2 oder 1 Gew.-% der kristallinen Modifikation II der Verbindung der Formel (I) bei der Formulierung eingesetzt.

Bevorzugt sind auch Zusammensetzungen, die von etwa 80 bis etwa 100 Gew.-% der kristallinen Modifikation I der Verbindung der Formel (I) und von etwa 20 bis etwa 0 Gew.-% der kristallinen Modifikation II der Verbindung der Formel (I) enthalten. Besonders bevorzugt sind Zusammensetzungen, die von etwa 85 Gew.-% bis etwa 100 Gew.-% der kristallinen Modifikation I und von etwa 15 Gew.-% bis etwa 0 Gew.-% der kristallinen Modifikation II enthalten. Ganz besonders bevorzugt sind Zusammensetzungen, die von etwa 90 Gew.-% bis etwa 100 Gew.-% der kristallinen Modifikation I und von etwa 10 Gew.-% bis etwa 0 Gew.-% der kristallinen Modifikation II enthalten. Insbesondere bevorzugt sind Zusammensetzungen, die von etwa 95 Gew.-% bis etwa 100 Gew.-% der kristallinen Modifikation I und von etwa 5 Gew.-% bis etwa 0 Gew.-% der kristallinen Modifikation II enthalten. Am meisten bevorzugt sind Zusammensetzungen, die von etwa 96 Gew.-% bis etwa 100 Gew.-% der kristallinen Modifikation I und von etwa 4 Gew.-% bis etwa 0 Gew.-% der kristallinen Modifikation II oder von etwa 97 Gew.-% bis etwa 100 Gew.-% der kristallinen Modifikation I und von etwa 3 Gew.-% bis etwa 0 Gew.-% der kristallinen Modifikation II oder von etwa 98 Gew.-% bis etwa 100 Gew.-% der kristallinen Modifikation I und von etwa 2 Gew.-% bis etwa 0 Gew.-% der kristallinen Modifikation II oder von 99 Gew.-% bis etwa 100 Gew.-% der kristallinen Modifikation I und von etwa 1 Gew.-% bis etwa 0 Gew.-% der kristallinen Modifikation II enthalten.

Ein weiterer Gegenstand der Erfindung sind Mittel zur Bekämpfung von Schädlingen, die die Verbindung der Formel (I) in der kristallinen Modifikation I und mindestens eine Form der Verbindung der Formel (I) enthalten, die sich von der kristallinen Modifikation I unterscheidet. Beispiele für die Form der Verbindung der Formel (I), die sich von der kristallinen Modifikation I unterscheidet, sind die kristalline Modifikation II oder die amorphe Form. Bevorzugt wird eine Wirkstoffqualität bei der Formulierung eingesetzt, die weniger als 20 Gew.-% der Form der Verbindung der Formel (I), die sich von der kristallinen Modifikation I der Verbindung der Formel (I) unterscheidet, enthält, besonders bevorzugt weniger als 10 Gew.-%, ganz besonders bevorzugt weniger als 5 Gew.-% und am meisten bevorzugt weniger als 2 Gew.-% der Form der Verbindung der Formel (I), die sich von der kristallinen Modifikation I der Verbindung der Formel (I) unterscheidet.

Aufgrund ihrer Stabilität eignet sich die kristalline Modifikation I der Verbindung der Formel (I) ganz allgemein als Ausgangsmaterial für die Herstellung jedweder die Verbindung der Formel (I) enthaltender Mittel zur Bekämpfung von Schädlingen, auch wenn die Verbindung der Formel (I) nach der Formulierung nicht mehr in der kristallinen Modifikation (I), sondern etwa in gelöster Form vorliegt.

Gegenstand der Erfindung sind daher ferner auch Verfahren zur Herstellung von Mitteln zur Bekämpfung von Schädlingen, welche die kristalline Modifikation I der Verbindung der Formel (I) verwenden sowie die Verbindung der Formel (I) enthaltende Mittel zur Bekämpfung von Schädlingen, die aus der kristallinen Modifikation I der Verbindung der Formel (I) erhalten wurden. Durch den Einsatz der kristallinen Modifikation I der Verbindung der Formel (I) wird die Sicherheit für Zubereitungen der Verbindung der Formel (I) erhöht und somit das Risiko falscher Dosierungen verringert.

Die kristalline Modifikation I der Verbindung der Formel (I) kann in bekannter Weise in die üblichen Formulierungen überführt werden, wie Suspensionskonzentrate, Öl basierte Suspensionskonzentrate, Kolloidale Konzentrate, Dispergierbare Konzentrate, Emulgierbare Konzentrate (Emulsionskonzentrate), Emulsionsbeizen, Suspensionsbeizen, Granulate, Mikrogranulate, Suspoemulsionen, Wasserlösliche Granulate, Wasserlösliche Konzentrate und Wasserdispergierbare Granulate, unter Verwendung geeigneter Hilfs- und Trägerstoffe oder Lösemittel. Hierbei soll die wirksame Verbindung in einer Konzentration von etwa 0,5 bis 90 Gew.-% der Gesamtmischung vorhanden sein, d.h. in Mengen, die ausreichend sind, um den notwendigen Dosierungsspiegel zu erreichen. Die Formulierungen werden beispielsweise hergestellt durch Verstrecken der kristallinen Modifikation I der Verbindung der Formel (I) mit Wasser, Lösemitteln und/oder Trägerstoffen, gegebenenfalls unter Verwendung von Emulgier- und/oder Dispergiermitteln, und/oder anderen Hilfsstoffen, wie z.B. Penetrationshilfsmitteln.

Bei der Herstellung von Suspensionskonzentraten, auch von solchen, die zur Saatgutbehandlung verwendet werden, werden im Allgemeinen neben dem Wirkstoff und einem Streckmittel (Wasser, Lösungsmittel oder Öl) weitere Hilfsmittel zugegeben. Zur Befeuchtung des Wirkstoffs in der kontinuierlichen Phase wird ein Netzmittel eingesetzt, zur Stabilisierung der Suspension in der flüssigen Phase werden Dispergierhilfsmittel verwendet, zum Emulgieren der nicht wässerige Phase werden bei Lösungsmittel oder Öl enthaltenden Suspensionskonzentraten Emulgiermittel eingesetzt. Sofern notwendig werden Frostschutzmittel, Biozide, Verdickungsmittel, Farbstoffe Spreizmittel oder / und Aufnahmeförderer eingearbeitet.

Die kristalline Modifikation I der Verbindung der Formel (I) kann durch die im Folgenden beschriebenen Verfahren erhalten werden.

Die Verbindung der Formel (I) in der kristallinen Modifikation II (4-{[(6-Chlorpyrid-3-yl)methyl](methyl)amino}furan-2(5H)-on, bekannt aus EP-A 0 539 588; metastabile Modifikation) wird nach Zugabe eines geeigneten Lösungsmittels, in dem die Verbindung der Formel (I) in der kristallinen Modifikation II in Form einer Suspension vorliegt, unter Rühren auf ca. 80 °C erwärmt (Tempern) und ca. eine bis mehrere Stunden weiter bei dieser Temperatur gerührt. Über Nacht wird die Probe im unbeheizten Block belassen. Man erhält die Verbindung der Formel (I) in der kristallinen Modifikation I (stabile Modifikation).

In einem alternativen Verfahren wird die Verbindung der Formel (I) in der kristallinen Modifikation II (4-{[(6-Chlorpyrid-3-yl)methyl](methyl)amino}furan-2(5H)-on, bekannt aus EP-A 0 539 588; metastabile Modifikation) unter Rühren mit einem geeigneten Lösungsmittel, z.B. Essigsäurebutylester oder Toluol, aufgekocht und 1-12 h bei Rückflusstemperatur (90-120 °C) nachgerührt. Anschließend stellt man die Heizung ab, lässt unter Rühren abkühlen und setzt bei ca. 90-110 °C etwas Verbindung der Formel (I) in der kristallinen Modifikation I (stabile Modifikation; erhältlich gemäß Beispiel 1) zu. Nach Erreichen von Raumtemperatur saugt man ab, wäscht mit einem geeigneten Lösungsmittel, z.B. Petrolether, Toluol, Hexan, Heptan oder Cyclohexan, nach und trocknet den Rückstand bei 30-60°C im Vakuumtrockenschrank. Man erhält zu 90-95 % die Verbindung der Formel (I) in der kristallinen Modifikation I (stabile Modifikation) und zu 5-10 % die Verbindung der Formel (I) in der kristallinen Modifikation II (metastabile Modifikation).

Spezielle Ausführungsformen der oben beschriebenen Verfahren zur Herstellung der kristallinen Modifikation I der Verbindung der Formel (I) sind in den Beispielen 1, 1.1 und 1.2 beschrieben.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sen-sible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora e-rythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dic-tyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie *N*-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile oder Saatgut, gemischt oder verbunden sind. Der feste oder flüssige Trägerstoff ist im Allgemeinen inert und sollte in der Landwirtschaft verwendbar sein.

Als feste oder flüssige Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Der Wirkstoffgehalt der aus den Formulierungen bereiteten Anwendungsformen kann in weiten Bereichen variieren. Die Wirkstoffkonzentration der Anwendungsformen liegt im Bereich von 0,00000001 bis 97 Gew.-% Wirkstoff, vorzugsweise im Bereich von 0,0000001 bis 97 Gew.-%, besonders bevorzugt im Bereich von 0,000001 bis 83 Gew.-% oder 0,000001 bis 5 Gew.-% und ganz besonders bevorzugt im Bereich von 0,0001 bis 1 Gew.-%.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden.

Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch einoder mehrschichtiges Umhüllen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kemfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und - plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Cherropodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-For-mulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryI-IIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe erfolgt im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pouron und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung in der Tierhaltung, beispielsweise Haltung von Vieh, Geflügel, Haustiere können die Wirkstoffe oder Zusammensetzungen als geeignete Formulierungen, beispielsweise Pulver, Emulsionen, fließfähige Mittel verwendet werden. Gewöhnlicherweise enthalten geeignete Formulierungen die Wirkstoffe in einer Konzentration im Bereich von 0,1 bis 80 Gew.-%, bevorzugt im Bereich von 1- 60 Gew.-%, besonders bevorzugt im Bereich von 5 bis 30 Gew.-% enthalten. Die Formulierungen können direkt oder verdünnt, bevorzugt nach 100- bis 10 000-facher Verdünnung angewendet werden. Die Wirkstoffe bzw. die Zusammensetzungen können auch als chemisches Bad verwendet werden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Beschreibung der Abbildungen

- Figur 1:: Röntgen-Pulverdiffraktogramm der kristallinen Modifikation I der Verbindung der Formel (I)
- Figur 2:: Röntgen-Pulverdiffraktogramm der kristallinen Modifikation II der Verbindung der Formel (I)
- Figur 3:: IR Spektrum der kristallinen Modifikation I der Verbindung der Formel (I)
- Figur 4:: IR Spektrum der kristallinen Modifikation II der Verbindung der Formel (I)
- Figur 5:: Raman Spektrum der kristallinen Modifikation I der Verbindung der Formel (I)
- Figur 6:: Raman Spektrum der kristallinen Modifikation II der Verbindung der Formel (I)

Die folgenden Beispiele erläutern die Erfindung ohne sie einzuschränken. Die in den folgenden Beispielen eingesetzten Lösungsmittelsysteme sind besonders bevorzugt.

### Beispiel 1

### Herstellung der Verbindung der Formel (I) in der kristallinen Modifikation I

120 mg der Verbindung der Formel (I) in der kristallinen Modifikation II (4-{[(6-Chlorpyrid-3-yl)methyl](methyl)amino}furan-2(5H)-on, bekannt aus EP-A 0 539 588; metastabile Modifikation) werden nach Zugabe von 4 Tropfen dest. Wasser unter Rühren auf 80°C erwärmt und ca. 3 Stunden weiter bei dieser Temperatur gerührt. Über Nacht wird die Probe im unbeheizten Block belassen. Man erhält die Verbindung der Formel (I) in der kristallinen Modifikation I (stabile Modifikation).

### Beispiel 1.1

### Herstellung der Verbindung der Formel (I) in der kristallinen Modifikation I in Gegenwart eines Verdünnungsmittels

200 g Verbindung der Formel (I) in der kristallinen Modifikation II (4-{[(6-Chlorpyrid-3-yl)methyl](methyl)amino}furan-2(5H)-on, bekannt aus EP-A 0 539 588; metastabile Modifikation) wurden unter Rühren mit 400 ml Essigsäurebutylester aufgekocht und eine Stunde bei Rückflusstemperatur (120 °C) nachgerührt. Anschließend stellt man die Heizung ab, lässt unter Rühren abkühlen und setzt bei ca. 100 °C etwas Verbindung der Formel (I) in der kristallinen Modifikation 1 (stabile Modifikation; vgl. Beispiel 1) zu.

Nach Erreichen von Raumtemperatur saugt man ab, wäscht mit 250 ml Petrolether nach und trocknet den Rückstand bei 45 °C im Vakuumtrockenschrank.

Man erhält 189,1 g eines Feststoffs (HPLC 98,0 %) der zu 90-95 % Verbindung der Formel (I) in der kristallinen Modifikation I (stabile Modifikation) und zu 5-10 % Verbindung der Formel (I) in der kristallinen Modifikation II (metastabile Modifikation) enthält.

### Beispiel 1.2

### Herstellung der Verbindung der Formel (I) in der kristallinen Modifikation I ohne Verdünnungsmittel

4,5 g Verbindung der Formel (I) in der kristallinen Modifikation II (4-{[(6-Chlorpyrid-3-yl)methyl](methyl) amino}furan-2(5H)-on, bekannt aus EP-A 0 539 588; metastabile Modifikation) werden im Ölbad bei 125 °C aufgeschmolzen, auf 105 °C abgekühlt, mit Impfkristall (Verbindung der Formel (I) in der kristallinen Modifikation I; stabile Modifikation; vgl. Beispiel 1) versetzt und die Schmelze auf Raumtemperatur abgekühlt.

Man erhält 4,4 g Kristalle; 85-90 % Verbindung der Formel (I) in der kristallinen Modifikation I (stabile Modifikation), 10-15 % Verbindung der Formel (I) in der kristallinen Modifikation II (metastabile Modifikation).

### Beispiel 2

### Herstellung eines Suspensionskonzentrats

Zur Herstellung eines Suspensionskonzentrates werden zunächst alle flüssigen Komponenten miteinander vermischt. Im nächsten Schritt werden die Feststoffe zugegeben und so lange gerührt, bis eine homogene Suspension entsteht. Die homogene Suspension wird zunächst einer Grob- und dann einer Feinmahlung unterworfen, so dass man eine Suspension erhält, in der 90% der Feststoffpartikel eine Teilchengröße unterhalb von 10 µm aufweisen. Bei der Mahlung ist es von Bedeutung dass der Druck über der Mühle konstant bleibt und dass die Temperatur nicht über 40°C ansteigt, um eine Festmahlung zu vermeiden. Anschließend fügt man unter Rühren bei Raumtemperatur Kelzan® S und Wasser hinzu. Es wird ein homogenes Suspensionskonzentrat erhalten.

### Beispiel 2.1

### Herstellung eines Suspensionskonzentrats der kristallinen Modifikation I

Zur Herstellung eines Suspensionskonzentrates der kristallinen Modifikation I der Verbindung der Formel (I) werden
43,3 g kristalline Modifikation I (> 95 %) der Formel (I)
8,0 g Soprophor TS 29
2,0 g Atlox 4913
20 g Glycerin
0,8 g Kelzan S
0,16 g Preventol D7
0,240 g Proxel GXL
0,2 g Silicon Antischaumemulsion SRE, und
145,3 g Wasser
wie beschrieben verarbeitet.

### Beispiel 2.2

### Herstellung eines Suspensionskonzentrats der kristallinen Modifikation II / Vergleichsformulierung

Zur Herstellung eines Suspensionskonzentrates der kristallinen Modifikation II der Verbindung der Formel (I) werden
43,6 g kristalline Modifikation II (> 95 %) der Formel (I)
8,0 g Soprophor TS 29
2,0 g Atlox 4913
20 g Glycerin
0,8 g Kelzan S
0,16 g Preventol D7
0,240 g Proxel GXL
0,2 g Silicon Antischaumemulsion SRE, und
145,0 g Wasser
wie beschrieben verarbeitet.

### Beispiel 2.3

### Herstellung eines Öl-basierten Suspensionskonzentrats (OD) der kristallinen Modifikation I

Zur Herstellung eines Öl-basierten Suspensionskonzentrates (OD) der kristallinen Modifikation I der Verbindung der Formel (I) in einer Konzentration von 100g Wirkstoff/1 (OD 100 Formulierung) werden

| | | |
|---|---|---|
| 298 | g | kristalline Modifikation I (> 85 %) der Formel (I) |
| 295 | g | Arlaton T(V) |
| 132,8 | g | Atlox 4894 |
| 14,7 | g | Morwet D 425 |
| 1,5 | g | Silfoam SC 1132 |
| 5,9 | g | wasserfreie Zitronensäure |
| 5,9 | g | Vulkanox BHT |
| unter Rühren bei Raumtemperatur in ein Gemisch aus | | |
| 590 | g | der Verbindung der Formel (II) |
| 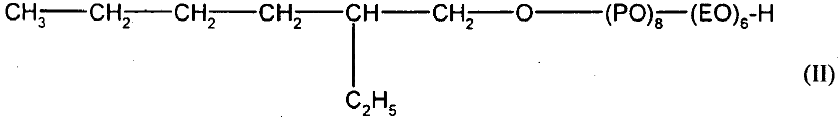 | | |
| in welcher | | |
| EO | | für -CH₂-CH₂-O- steht, |
| | | für 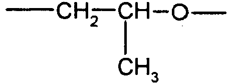 steht und |
| PO | | |
| die Zahlen 8 und 6 Durchschnittswerte darstellen, und | | |
| 1606 | g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen.

Die durch ihre Handelsnamen definierten Komponenten der erfindungsgemäßen Zusammensetzungen sind bei folgenden Lieferanten erhältlich:

| | |
|---|---|
| Handelsname | Lieferant |
| Arlaton T(V) | Uniqema |
| Atlox 4913 | Uniqema |
| Atlox 4894 | Uniqema |
| Kelzan S | CP Kelco |
| Morwet D 425 | Akzo Nobel |
| Preventol D7 | Bayer AG |
| Proxel GXL | Bayer AG |
| Silfoam SC 1132 | Wacker Silicones |
| Silicon Antischaumemulsion SRE | Wacker Silicones |
| Soprophor TS 29 | Rhodia |
| Vulkanox BHT | Bayer AG |

### Beispiel 3

### Herstellungseigenschaften

Die Herstellung der Formulierung des Beispiels 2.1 auf einem Dispermat^{®} SL der Firma Getzmann GmbH verlief nach Standard-Einstellungen; 0,75 bis 1,0 mm Glassperlen, 80% Füllgrad, 4000 Umdrehungen pro Sekunde, Durchsatz 0,3 L / Stunde, Maximale Temperatur in der Mühle 36°C bei einem Druck < 0,01 bar.

Bei der Herstellung der Formulierung des Beispiels 2.2 auf einem Dispermat^{®} SL musste der Durchsatz zur Vermeidung einer Festmahlung auf 0,1 L / Stunde reduziert werden. Aufgrund von stark steigender Temperatur und steigendem Druck wäre es nicht möglich gewesen, die Formulierung von Beispiel 2.2 ohne Reduzierung des Durchsatzes herzustellen.

### Beispiel 4

### Lagerungseigenschaften

Die Formulierungen der Beispiele 2.1 und 2.2 wurden 8 Wochen bei 54°C eingelagert. Nach der Lagerung wurde die Probe auf Raumtemperatur gebracht und die Eigenschaften der gelagerten Formulierungen mit denen von frisch gemäß Beispiel 2 hergestellten Proben verglichen.

Die Formulierung aus Beispiel 2.1 zeigte eine leichte Phasentrennung war aber gut re-dispergierbar. Die formuliertechnischen Eigenschaften der Formulierung waren nach Lagerung unverändert.

Die Vergleichsformulierung aus Beispiel 2.2 zeigte eine leichte Phasentrennung welche nach intensivem Schütteln re-dispergierbar war. Weiter wurde ein Kristallwachstum und ein starkes Agglomerieren der Einzelpartikel beobachtet. Die aus dieser Formulierung hergestellte 1%ige wässrige Spritzbrühe sedimentierte innerhalb einer Stunde und war deshalb schlecht applizierbar.

### Biologisches Beispiel

Wirkung der kristallinen Modifikation I der Verbindung der Formel (I) gegen pflanzenschädigende Insekten / Feldversuch Reis, Philippinen

### N ilaparvata lugens - Test (Blattapplikation)

Zur Herstellung einer zweckmäßigen Wirkstoffzubereitung verdünnt man das nach Beispiel 2.3 hergestellte Öl-basierte Suspensionskonzentrat (OD 100 Formulierung) mit Wasser auf die gewünschte Konzentration. Nach der gewünschten Zeit wird die Abtötung in % bestimmt. Dabei bedeutet 100 %, dass alle Zikaden abgetötet wurden; 0 % bedeutet, dass keine Zikaden abgetötet wurden.

In diesem Test zeigt die Verbindung der Formel (I) in der kristallinen Modifikation I gute Wirksamkeit:

| | |
|---|---|
| Wirkstoffkonzentration in g/ha: | 200 |
| Abtötungsgrad in % nach 14 Tagen: | 100 |

## Patentansprüche

1. Kristalline Modifikation I der Verbindung der Formel (I) 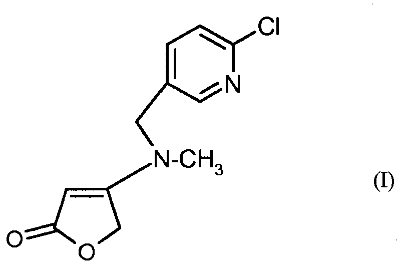 **dadurch gekennzeichnet, dass** ihr Röntgen-Pulverdiffraktogramm bei Verwendung von Cu Kα-Strahlung der Wellenlänge 1.540598 Cu folgende Reflexlagen aufweist
| **2Theta / °** |
|---|
| 16.80 |
| 19.58 |
| 21.11 |
| 21.32 |
| 23.23 |
| 28.00 |

2. Kristalline Modifikation I gemäß Anspruch 1, **dadurch gekennzeichnet, dass** ihr Röntgen-Pulverdiffraktogramm bei Verwendung von Cu Kα-Strahlung folgende Reflexlagen aufweist
| **2Theta / °** |
|---|
| 16.80 |
| 19.58 |
| 21.11 |
| 21.32 |
| 22.92 |
| 23.23 |
| 23.97 |
| 28.00 |

3. Zusammensetzung umfassend die kristalline Modifikation I der Verbindung der Formel (I) nach einem oder mehreren der Ansprüche 1 bis 2 und geeignete Hilfsstoffe.

4. Zusammensetzung umfassend die kristalline Modifikation I der Verbindung der Formel (I) nach einem oder mehreren der Ansprüche 1 bis 2 und die kristalline Modifikation II der Verbindung der Formel (I) und geeignete Hilfsstoffe, **dadurch gekennzeichnet, dass** die Zusammensetzung nicht mehr als 20 Gew. % der kristallinen Modifikation II der Verbindung der Formel (I) enthält.

5. Verwendung der Verbindung der Formel (I) der kristallinen Modifikation I nach einem oder mehreren der Ansprüche 1 bis 2 oder der Zusammensetzung nach Anspruch 3 oder 4 zur Herstellung von Mitteln zur Bekämpfung von Schädlingen.

6. Verwendung der Verbindung der Formel (I) der kristallinen Modifikation I nach einem oder mehreren der Ansprüche 1 bis 2 oder der Zusammensetzung nach Anspruch 3 oder 4 zur Bekämpfung von Pflanzenschädlingen.

7. Verbindung der Formel (I) der kristallinen Modifikation I nach einem oder mehreren der Ansprüche 1 bis 2 oder Zusammensetzung nach Anspruch 3 oder 4 zur Bekämpfung von tierischen Parasiten.

8. Verwendung der Verbindung der Formel (I) der kristallinen Modifikation I nach einem oder mehreren der Ansprüche 1 bis 2 oder der Zusammensetzung nach Anspruch 3 oder 4 zur Bekämpfung von Insekten, die technische Materialien zerstören.

9. Verwendung der Verbindung der Formel (I) der kristallinen Modifikation I nach einem oder mehreren der Ansprüche 1 bis 2 oder der Zusammensetzung nach Anspruch 3 oder 4 zur Behandlung von Saatgut.

## Claims

1. Crystalline modification I of the compound of the formula (I) 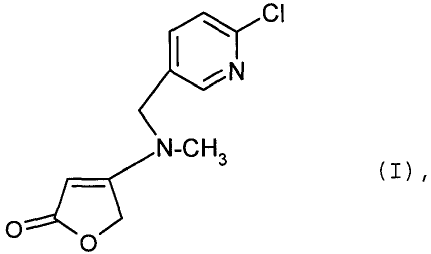 **characterized in that** its x-ray powder diffractogram, when using Cu Kα radiation of wavelength 1.540598 Cu, has the following reflection planes
| **2 Theta / °** |
|---|
| 16.80 |
| 19.58 |
| 21.11 |
| 21.32 |
| 23.23 |
| 28.00 |

2. Crystalline modification I according to Claim 1, **characterized in that** its x-ray powder diffractogram, when using Cu Kα radiation, has the following reflection planes
| **2 Theta / °** |
|---|
| 16.80 |
| 19.58 |
| 21.11 |
| 21.32 |
| 22.92 |
| 23.23 |
| 23.97 |
| 28.00 |

3. Composition comprising the crystalline modification I of the compound of the formula (I) according to one or more of Claims 1 and 2 and suitable auxiliaries.

4. Composition comprising the crystalline modification I of the compound of the formula (I) according to one or more of Claims 1 and 2 and the crystalline modification II of the compound of the formula (I) and suitable auxiliaries, **characterized in that** the composition comprises not more than 20% by weight of the crystsalline modification II of the compound of the formula (I).

5. Use of the compound of the formula (I) of the crystalline modification I according to one or more of Claims 1 and 2 or of the composition according to Claim 3 or 4 for preparing compositions for controlling pests.

6. Use of the compound of the formula (I) of the crystalline modification I according to one or more of Claims 1 and 2 or of the composition according to Claim 3 or 4 for controlling pests of plants.

7. Compound of the formula (I) of the crystalline modification I according to one or more of Claims 1 and 2 or composition according to Claim 3 or 4 for controlling animal parasites.

8. Use of the compound of the formula (I) of the crystalline modification I according to one or more of Claims 1 and 2 or of the composition according to Claim 3 or 4 for controlling insects which destroy industrial materials.

9. Use of the compound of the formula (I) of the crystalline modification I according to one or more of Claims 1 and 2 or of the composition according to Claim 3 or 4 for treating seed.

## Revendications

1. Forme cristalline I du composé de formule (I) 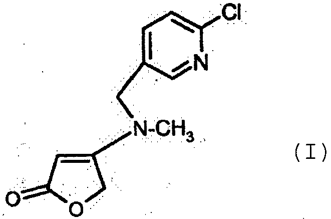 **caractérisée en ce que** son diagramme de diffraction des rayons X sur poudre avec utilisation de la raie Kα du cuivre de longueur d'onde 1,540598 Cu présente les réflexions suivantes
| **2 thêta/°** |
|---|
| 16,80 |
| 19,58 |
| 21,11 |
| 21,32 |
| 23,23 |
| 28,00 |

2. Forme cristalline I selon la revendication 1, **caractérisée en ce que** son diagramme de diffraction des rayons X sur poudre avec utilisation de la raie Kα du cuivre présente les réflexions suivantes
| **2 thêta/°** |
|---|
| 16,80 |
| 19,58 |
| 21,11 |
| 21,32 |
| 22, 92 |
| 23,23 |
| 23,97 |
| 28,00 |

3. Composition comprenant la forme cristalline I du composé de formule (I) selon une ou plusieurs des revendications 1 et 2, et des adjuvants appropriés.

4. Composition comprenant la forme cristalline I du composé de formule (I) selon une ou plusieurs des revendications 1 et 2 et la forme cristalline II du composé de formule (I) et des adjuvants appropriés, **caractérisée en ce que** la composition ne contient pas plus de 20 % en poids de la forme cristalline II du composé de formule (I).

5. Utilisation du composé de formule (I) de forme cristalline I selon une ou plusieurs des revendications 1 et 2 ou de la composition selon la revendication 3 ou 4, pour la préparation de produits destinés à la lutte contre des nuisibles.

6. Utilisation du composé de formule (I) de forme cristalline I selon une ou plusieurs des revendications 1 et 2 ou de la composition selon la revendication 3 ou 4, pour la lutte contre des nuisibles pour les plantes.

7. Composé de formule (I) de forme cristalline I selon une ou plusieurs des revendications 1 et 2 ou composition selon la revendication 3 ou 4, destinés à la lutte contre des parasites animaux.

8. Utilisation du composé de formule (I) de forme cristalline I selon une ou plusieurs des revendications 1 et 2 ou de la composition selon la revendication 3 ou 4, pour la lutte contre des insectes qui détruisent des matériaux industriels.

9. Utilisation du composé de formule (I) de forme cristalline I selon une ou plusieurs des revendications 1 et 2 ou de la composition selon la revendication 3 ou 4, pour le traitement de semences.
